# EUROPEAN PATENT SPECIFICATION

(11) **EP 2 539 441 B1**
(45) Date of publication and mention of the grant of the patent: **28.09.2016**
(21) Application number: 11704979.1
(22) Date of filing: 21.02.2011
(51) Int. Cl.: C12N 5/078, C12N 5/0789

(54) **CELL CULTURE MEDIUM FOR THE GROWTH AND DIFFERENTIATION OF CELLS OF THE HEMATOPOIETIC LINEAGE**
ZELLKULTURMEDIUM FÜR DAS WACHSTUM UND DIE DIFFERENZIERUNG VON HEMATOPOIETISCHEN ZELLEN
MILIEU DE CULTURE POUR LA CROISSANCE ET DIFFÉRENCIATION DE CELLULES HÉMATOPOÏÉTIQUES

(30) Priority: 22.02.2010 US 306682 P
(43) Date of publication of application: 02.01.2013
(73) Proprietor: Université Pierre et Marie Curie (Paris 6), 75005 Paris (FR); Etablissement Français du Sang, 93210 La Plaine Saint Denis (FR); Assistance Publique Hôpitaux De Paris, 75001 Paris (FR)
(72) Inventor: DOUAY, Luc, 75007 Paris (FR); GIARRATANA, Marie-Catherine, 93400 Saint Ouen (FR)
(74) Representative: Lavoix
(86) International application number: PCT/EP2011/052511
(87) International publication number: WO 2011/101468

(56) References cited:
- WO-A1-2005/056778
- WO-A1-2005/118780
- KR-A- 20080 037 281
- GIARRATANA M-C ET AL: "Ex vivo generation of fully mature human red blood cells from hematopoietic stem cells", NATURE BIOTECHNOLOGY, NATURE PUBLISHING GROUP, NEW YORK, NY, US, vol. 23, no. 1, 1 January 2005 (2005-01-01), pages 69-74, XP002334363, ISSN: 1087-0156, DOI: DOI:10.1038/NBT1047
- POLONI A ET AL: "The ex vivo expansion capacity of normal human bone marrow cellsis dependent on experimental conditions: role of the cell concentration, serum and CD34+ cell selection in stroma-free cultures", HEMATOLOGY AND CELL THERAPY, SPRINGER-VERLAG, PA, vol. 39, no. 2, 1 April 1997 (1997-04-01), pages 49-58, XP019371944, ISSN: 1279-8509
- KOESTENBAUER SONJA ET AL: "Protocols for hematopoietic stem cell expansion from umbilical cord blood", CELL TRANSPLANTATION, US, vol. 18, no. 10, 1 January 2009 (2009-01-01), pages 1059-1068, XP008126313, ISSN: 0963-6897, DOI: DOI:10.3727/096368909X471288
- DOUAY LUC ET AL: "Ex vivo generation of human red blood cells: a new advance in stem cell engineering", METHODS IN MOLECULAR BIOLOGY, HUMANA PRESS INC, NJ, US, vol. 482, 1 January 2009 (2009-01-01), pages 127-140, XP009148257, ISSN: 1064-3745

## Description

### Field of the invention

The present invention relates to a cell culture medium for the growth and/or differentiation of cells of the hematopoietic lineage.

### Technical background

There is a continuing high demand of labile blood products, in particular for transfusion purpose, which is not satisfactorily fulfilled by the current supplies in natural human blood. As a consequence, numerous substitutes to natural blood have been explored.

However, stabilized or recombinant hemoglobins have shown disappointing performances, the indications of artificial oxygen transporters are limited and the development of "universal" red blood cells made compatible with the ABO system and/or the RhD antigen by enzymatic treatment or antigenic masking is slow. There is thus a need for alternatives to these methods.

In this regard, attempts to generate erythroid cells, such as red blood cells, from stem cells *in vitro,* is particularly favored.

However, it is a considerable challenge to reproduce *in vitro* what it takes nature several months to construct *in vivo.* In fact, in the course of its development in humans, erythropoiesis evolves from the mesoderm in two waves. Primitive erythropoiesis starts as early as the third week of gestation in the vitelline sac (extra-embryonic tissue) and gives rise to primitive nucleated erythrocytes, megaloblastic, which synthesize embryonic hemoglobin of the type Gower I(ζ2ε2) and Gower II (α2ε2). Definitive erythropoiesis starts during the fifth week of gestation in the aorta-gonad-mesonephros (AGM) region, before migrating to the fetal liver and then to the bone marrow. The erythroid cells produced mature little by little, leading to the production of enucleated red blood cells (RBC), normocytic and containing fetal (α2γ2) and then adult (α2β2) hemoglobin.

To date, several attempts at producing red blood cells from human embryonic stem cells have been reported, such as described by Ma et al. (2008) Proc. Natl. Acad. Sci. USA 105:13087-13092. However, these experiments generally rely on a coculture step in the presence of stromal cells, which renders scaling-up of the process difficult.

### Summary of the invention

The present invention arises from the unexpected finding, by the inventors, that a cell culture medium comprising insulin, transferrin and plasma or serum, was useful for the massive production of red blood cells or reticulocytes from human embryonic stem cells, human induced-Pluripotent Stem (iPS) cells, or human hematopoietic stem cells, without the requirement of a coculture on a cellular stroma.

Thus, the present invention relates to a cell culture medium for the growth and/or differentiation of cells of the hematopoietic lineage, comprising:
- insulin at a concentration of from 1 to 50 µg/ml;
- transferrin at a concentration of from 100 µg/ml to 2000 µg/ml; and
- plasma or serum at a concentration of from 1 % to 30%.

The present invention also relates to the use of a cell culture medium as defined above, for the growth and/or differentiation of cells of the hematopoietic lineage.

The present invention further relates to a method for growing and/or differentiating cells of the hematopoietic lineage comprising at least one step of culturing cells with a cell culture medium as defined above.

### Brief description of the figures

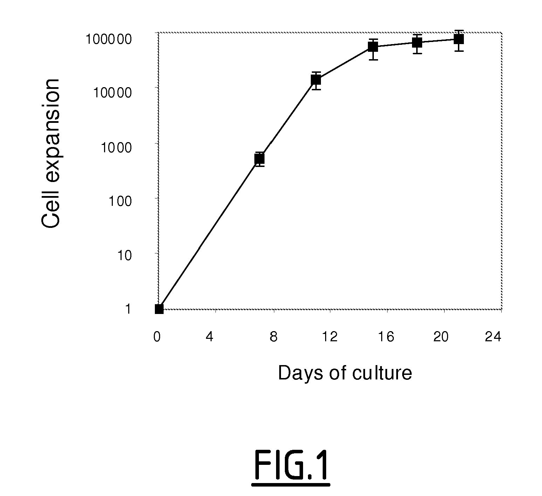

**Figure 1** shows expansion and differentiation of erythroid cells. Amplification of human CD34⁺ cells obtained by G-CSF mobilized leukapheresis (LK) and cultured in the presence of 5% human plasma according to a three-phase protocol (see Materials and Methods). Points are the mean values for four independent experiments.

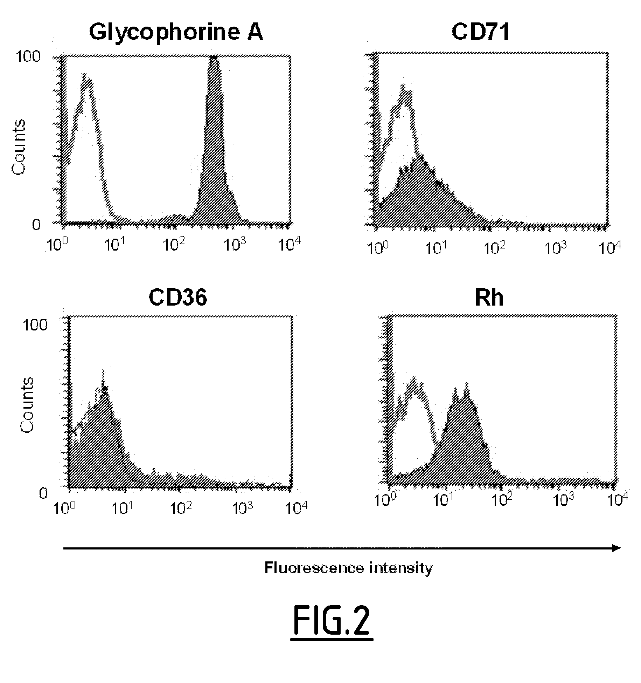

**Figure 2** shows flow cytometric analysis of CD71, CD36, glycophorin A and RhD expression on day 18. The solid histograms represent relevant mAbs and the open ones negative controls with irrelevant mAbs. This figure shows one experiment representative of four independent analyses.

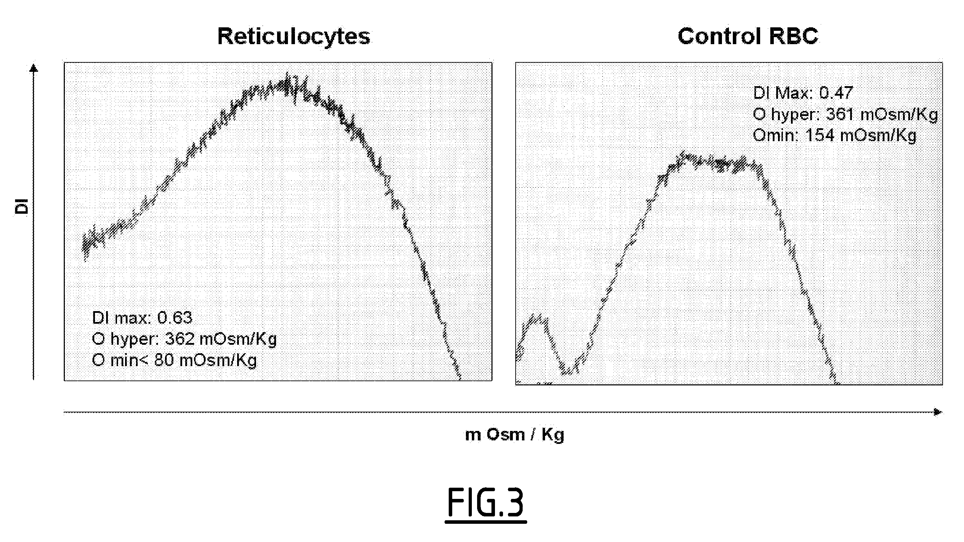

**Figure 3** shows the deformability profiles of LK derived reticulocytes on day 18 of culture (left) and control RBC (right). Ektacytometry in an osmolar gradient was used to measure the elongation of enucleated erythrocytes (see Materials and Methods). The curves define the osmoscan variables, i.e. the maximum deformability index (DIₘₐₓ) and O_{hyper} and Oₘᵢₙ, determined under isotonic, hypertonic and hypotonic conditions, respectively. Normal values of the variables were obtained by testing samples from 144 normal adults and ranged from 0.41 to 0.53 for DIₘₐₓ, from 143 to 163 mOsm/Kg for Oₘᵢₙ and from 335 to 375 mOsm/Kg for O_{hyper}.

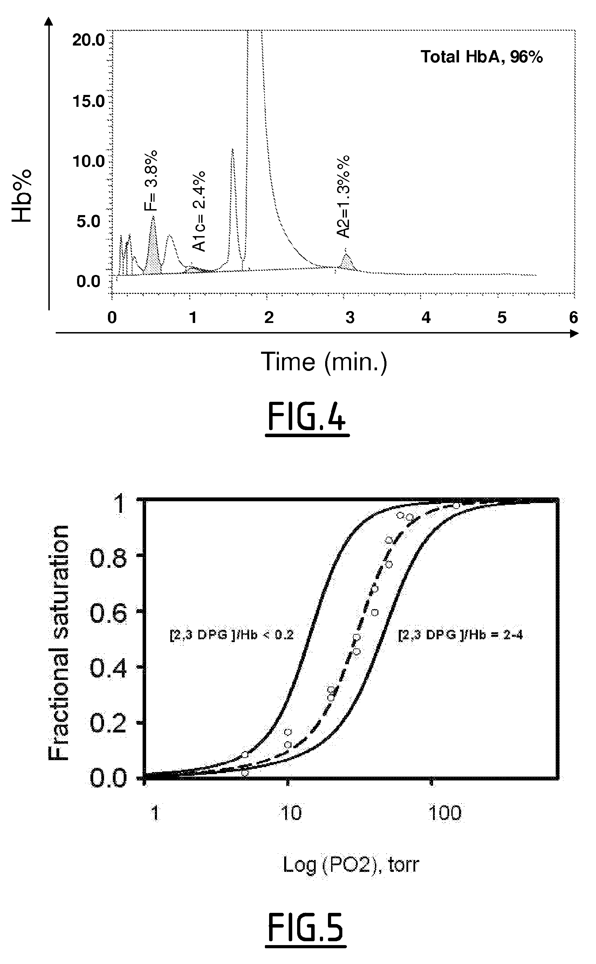

**Figure 4** shows the hemoglobin status of day 18 reticulocytes determined by HPLC analysis (Bio-Rad Variant II). The percentage of hemoglobin in the elution peak is indicated for the HbF, HbA1c, HbA2 and total HbA fractions.
**Figure 5** shows the tonometric oxygen binding curves at 37°C for a reticulocyte suspension (triangles) and a control RBC suspension at different DPG/Hb₄ ratios in 10 mM Hepes buffer (pH 7.4) containing 150 mM NaCl. The RBC isotherms were simulated from the average MWC parameters for 10 different blood samples.

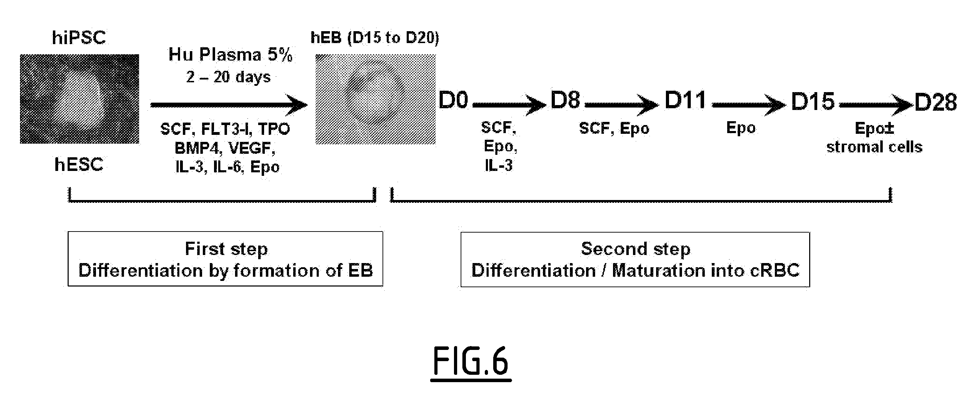

**Figure 6** shows a schematic representation of the successive culture steps used for production of erythroid cells from hESC. Clumps of undifferentiated hESC were cultured in "EB medium" for 20 days. Dissociated day 15 to day 20 EB were then cultured in a liquid medium for up to 28 days in the presence of sequential cocktails of cytokines (see Materials and Methods).

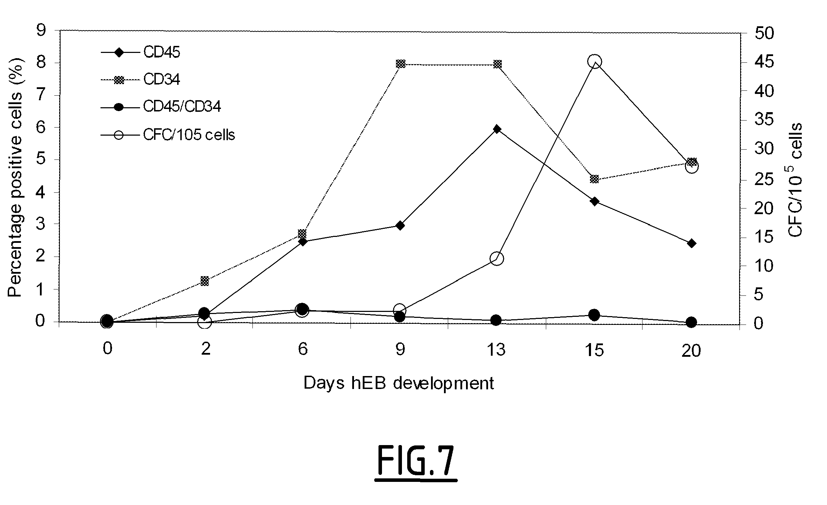

**Figure 7** shows the percentage expression of the hematopoietic markers CD45, CD34 and CD45/CD34 (left y-axis) of hESC-derived cells during EB differentiation and kinetics of CFC formation (right yaxis) in day 6 to day 20 EB (one representative experiment).

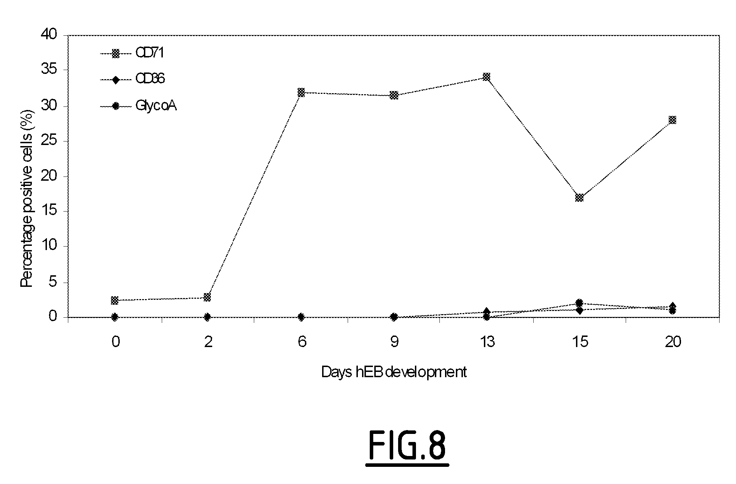

**Figure 8** shows the erythroid markers CD71, CD36 and GlycoA of hESC-derived cells during EB differentiation.

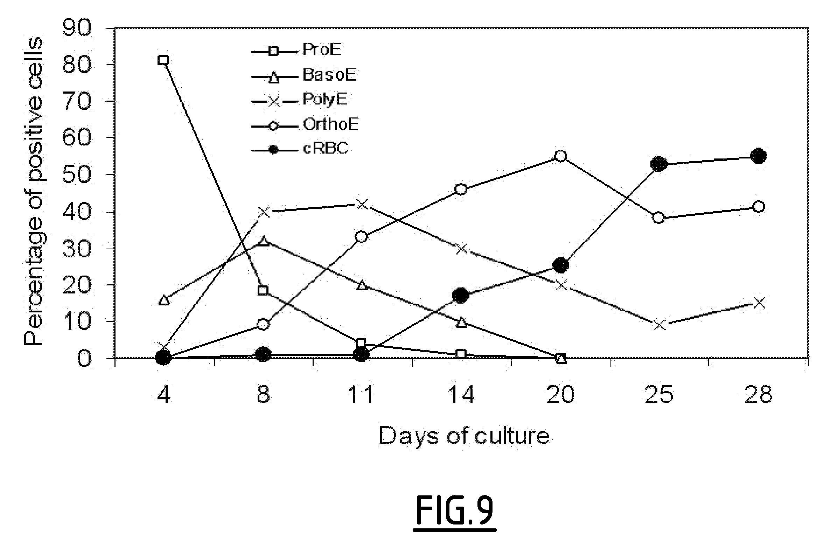

**Figure 9** shows the commitment to the erythroid lineage in liquid culture. Aliquots of the liquid cultures were taken at the indicated times for morphological analysis of the cells. Proerythroblasts (ProE); basophilic erythroblasts (BasoE); polychromatophilic erythroblasts (PolyE); orthochromatic erythroblasts (OrthoE); culturedred blood cells (cRBC). One representative experiment.

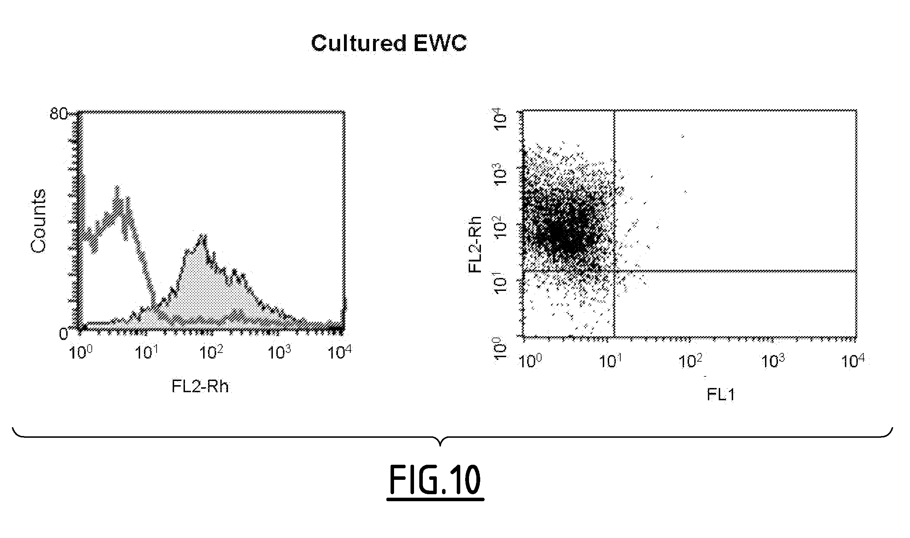

**Figure 10** shows the RhD antigen expression in cRBC derived from EWC. Enucleating cRBCs generated fromEBs culture were labeled with an anti-Rh D antibody (Biotest, Seraclone® Reagents for ABO Blood Typing). Cells are revealed with a secondary phycoerythrin-conjugated rabbit anti-human antibody (Beckman).

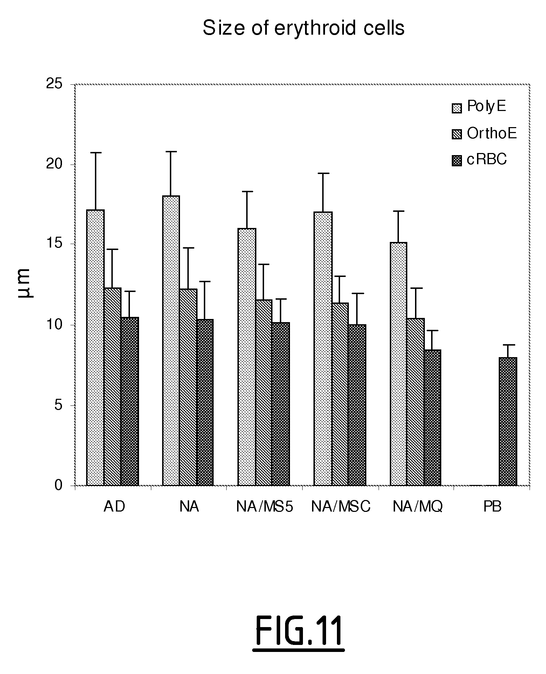

**Figure 11** shows the size of the erythroid cells under various culture conditions on day 25 of liquide culture. Cell size was measured with an optical micrometer in 100 cells in each case. AD (adherentcells); NA (non adherent cells); NA/MS5, NA/MSC, NA/MQ (non adherent cells coculturedon MS5 cells, MSC and macrophages respectively); PB (RBC from adult peripheral blood).

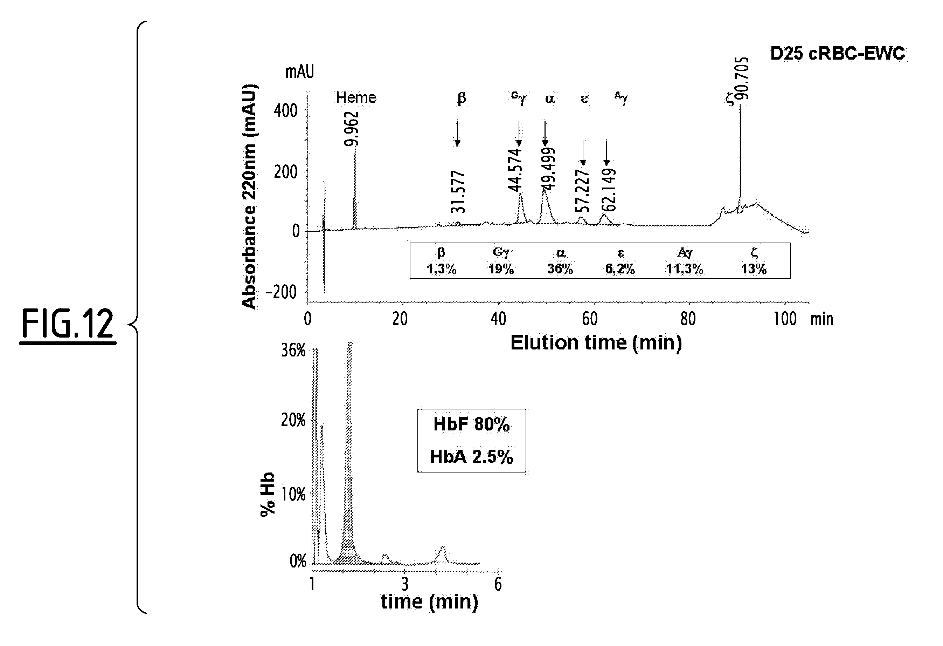

**Figure 12** shows representative RP-HPLC profiles of globin chains identified by mass spectrometry and HPLC analysis of the Hb, for day 25 cRBC derived from EWC.

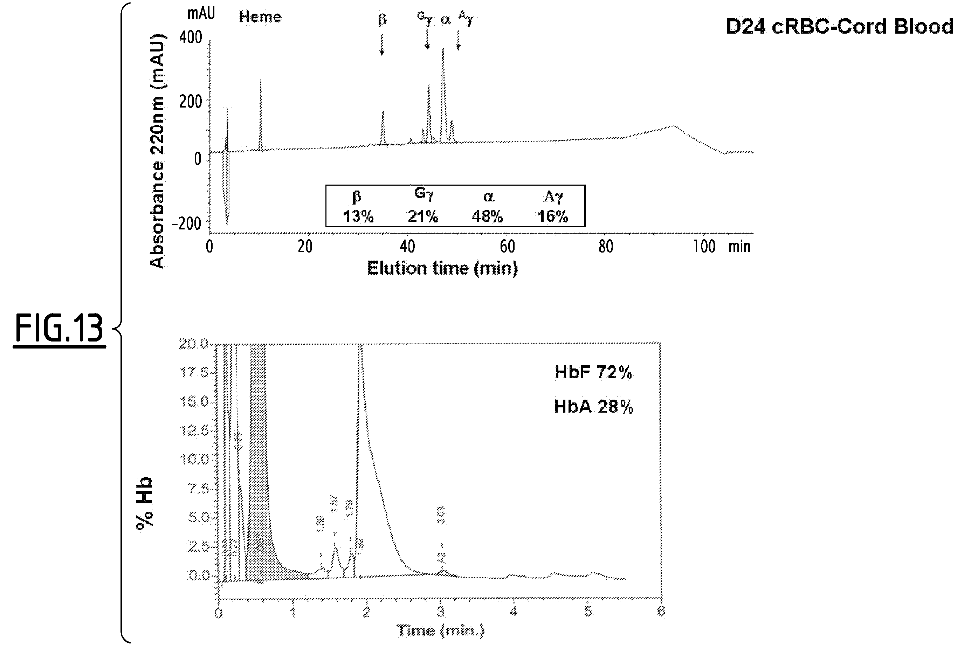

**Figure 13** shows representative RP-HPLC profiles of globin chains identified by mass spectrometry and HPLC analysis of the Hb, for day 24 cRBC derived from cord blood cells.

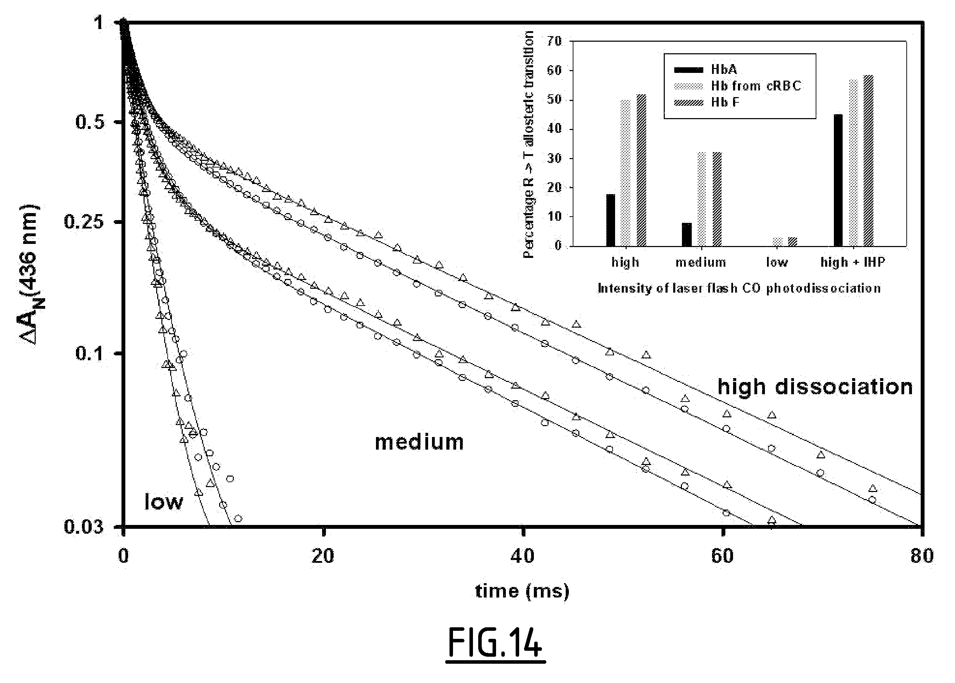

**Figure 14** shows CO rebinding kinetics after flash photolysis of cRBC hemoglobin (black curves with triangles) and hemoglobin from control native RBC (black curves with circles). The two samples show similar binding properties, including the allosteric transition. By varying the energy of the photolysis pulse, one can vary the total fraction of dissociated hemoglobin and thereby probe in detail the various partially bound populations. At high photolysis levels, more singly-bound tetramers are produced, which switch to the deoxy conformation (T-state)and rebind ligands more slowly. At intermediate levels (medium) one can better probe the doubly-bound tetramers, a form difficult to study by equilibrium techniques. At sufficiently low photolysis levels, the main photoproduct is triply-bound tetramers which rebind ligands rapidly (R-state). The percentage of R -> T allosteric transition is shown at different intensities of CO photo-dissociation. The CO rebinding kinetics can be simulated using two exponential terms for the fast rate inherent to the tetrameric species in the R-state conformation and the slow rate inherent to the T-state conformation. The R-state rate is typically 6x106/ms while the T-state rate is about 20 times slower. The fraction of T-state tetramers is much higher for HbF and Hb from cRBC-EWC as compared to HbA at different intensities of laser photo-dissociation, due to a shift of the allosteric transition. The increase inallosteric transition to the low affinity T-state tetramers upon addition of 1 mM IHP (inositol-hexa-phosphate) is larger for the HbA than that for HbF and Hb from cRBC-EWC. This can be explained by a lower affinity of HbF for IHP as already reported for 2,3 DPG and/or by the higher percentage of R-> T transition in the absence of allosteric effector for HbF as compared to HbA.

### Detailed description of the invention

As intended herein the expression "cells of the hematopoietic lineage" relates to cells to be found in the blood of mammals, in particular of humans, and to cells liable to yield such blood cells upon differentiation. More particularly, the expression "cells of the hematopoietic lineage" according to the invention relates to cells of the erythrocytic lineage, that is red blood cells (also called erythrocytes) and cells which are liable to yield red blood cells upon differentiation, either directly, *i.e.* in one step, or indirectly, *i.e.* in several steps. As is well-known to one of skill in the art, cells of the erythrocytic lineage notably comprise, classified by increasing degree of differentiation, embryonic stem cells, hematopoietic stem cells (HSCs), pro-erythroblasts, erythroblasts, reticulocytes, enucleated cells, in particular enucleated reticulocytes, and red blood cells. Cells of the hematopoietic lineage according to the invention thus notably encompass stem cells, in particular embryonic stem cells (ESC), adult stem cells, such as hematopoietic stem cells (HSCs), induced-pluripotent stem (iPS) cells, as well as embryoid bodies, but also pro-erythroblasts, erythroblasts, reticulocytes, and enucleated cells, in particular enucleated reticulocytes. Preferably, the cells of the hematopoietic lineage of the invention are human cells.

iPS cells are well-known to one of skill in the art. They can be obtained by numerous methods and from numerous cell types. By way of example, iPS cells can be obtained following the teachings of Takahashi & Yamanaka (2006) Cell 126:663-676 and Yamanaka et al. (2007) Nature 448:313-317

As intended herein, the term "growth" relates to the multiplication of cultured cells. As intended herein, the term "differentiation" relates to the acquisition by cells cultured in a culture medium of cellular characteristics which are not present in the cells initially used for seeding the cell culture medium. As intended herein "differentiation" notably denotes the acquisition of characteristics further committing the cells in the pathway towards differentiation into red blood cells. Thus, the cell culture medium of the invention is particularly useful for growing undifferentiated cells, such as embryonic stem cells, adult stem cells, such as hematopoietic stem cells, induced-pluripotent stem cells (iPS), or embryoid bodies, and differentiating them into reticulocytes, enucleated cells or red blood cells.

As intended herein the expression "cell culture medium" relates to any medium, in particular any liquid medium, liable to sustain the growth of eukaryotic cells, in particular mammalian cells, more particularly human cells.

Preferably, the cell culture medium of the invention is composed of a base culture medium to which is added:
- insulin at a concentration of from 1 to 50 µg/ml;
- transferrin at a concentration of from 100 µg/ml to 2000 µg/ml; and
- plasma or serum at a concentration of from 1 % to 30%.

Preferably, the base culture medium is liable by itself to generally sustain the growth of eukaryotic cells, in particular of mammalian cells, more particularly of human cells. Such base culture media are well known to one of skill in the art. By way of example, one may cite Iscove's Modified Dulbecco's Medium (IMDM) optionally complemented with glutamine or a glutamine-containing peptide. Thus, the cell culture medium according to the invention preferably further comprises Iscove's Modified Dulbecco's Medium (IMDM) optionally complemented with glutamine or a glutamine-containing peptide.

Preferably, insulin according to the invention is human recombinant insulin. Preferably also, insulin is at a concentration of from 5 µg/ml to 20 µg/ml, more preferably at a concentration of from 8 µg/ml to 12 µg/ml, and most preferably at a concentration of about 10 µg/ml.

Preferably, transferrin is human transferrin. Preferably, transferrin is iron-saturated. Preferably, also transferrin is at a concentration of from 200 µg/ml to 1000 µg/ml, more preferably at a concentration of from 300 µg/ml to 500 µg/ml, and most preferably at a concentration of about 330 µg/ml or 450 µg/ml. The transferrin may be recombinant.

Preferably plasma or serum is human plasma or serum. Preferably also, plasma or serum is at a concentration of from 1% to 20%, more preferably at a concentration of from 4% to 12%, even more preferably at a concentration of from 5% to 10%, and most preferably at a concentration of about 5% or 10%.

In an embodiment, the cell culture medium of the invention further comprises heparin, in particular at a concentration 0.5 UI/ml to 5 UI/ml, more particularly at a concentration of from 1.5 to 3.5 UI/ml, and most preferably at a concentration of about 2 UI/ml. Preferably, the cell culture medium of the invention comprises heparin when serum is also comprised in the cell culture medium.

In another embodiment, the cell culture medium of the invention further comprises erythropoietin (Epo), in particular human recombinant erythropoietin, preferably at a concentration of from 0.5 UI/ml to 20 UI/ml, more preferably at a concentration of from 2.5 UI/ml to 3.5 UI/ml, and most preferably at a concentration of about 3 UI/ml.

In another embodiment, the cell culture medium of the invention further comprises stem cell factor (SCF), in particular human recombinant stem cell factor, preferably at a concentration of from 50 ng/ml to 200 ng/ml, more preferably at a concentration of from 80 ng/ml to 120 ng/ml, and most preferably at a concentration of about 100 ng/ml.

In another embodiment, the cell culture medium of the invention further comprises interleukin-3 (IL-3), in particular human recombinant interleukin-3, preferably at a concentration of from 1 ng/ml to 30 ng/ml, more preferably at a concentration of from 4 ng/ml to 6 ng/ml, and most preferably at a concentration of about 5 ng/ml.

In a further embodiment, the cell culture medium according to the invention, further comprises hydrocortisone, preferably at a concentration of from 5.10⁻⁷ to 5.10⁻⁶ M, more preferably at a concentration of about 10⁻⁶ M.

In yet another embodiment, the cell culture medium according to invention further comprises at least one compound selected from:
- Thrombopoietin (TPO), in particular recombinant human thrombopoietin, preferably at a concentration of from 20 ng/ml to 200 ng/ml, more preferably at a concentration of from 80 ng/ml to 120 ng/ml, most preferably at a concentration of about 100 ng/ml;
- FMS-like tyrosine kinase 3 (FLT3) ligand, in particular recombinant human FLT3 ligand, preferably at a concentration of from 20 ng/ml to 200 ng/ml, more preferably at a concentration of from 80 ng/ml to 120 ng/ml, most preferably at a concentration of about 100 ng/ml;
- bone morphogenetic protein 4 (BMP4), in particular recombinant human bone morphogenetic protein 4, preferably at a concentration of from 1 ng/ml to 20 ng/ml, more preferably at a concentration of from 8 ng/ml to 12 ng/ml, most preferably at a concentration of about 10 ng/ml;
- vascular endothelial growth factor A165 (VEGF-A165), in particular recombinant human VEGF-A165, preferably at a concentration of from 1 ng/ml to 20 ng/ml, more preferably at a concentration of from 4 ng/ml to 6 ng/ml, most preferably at a concentration of about 5 ng/ml; and
- interleukin-6 (IL-6), in particular recombinant human IL-6, preferably at a concentration of from 1 ng/ml to 20 ng/ml, more preferably at a concentration of from 4 ng/ml to 6 ng/ml, most preferably at a concentration of about 5 ng/ml.

Preferably, the cell culture medium of the invention is used for growing hematopoietic stem cells (HSCs) and differentiating the HSCs into reticulocytes, enucleated cells, and/or red blood cells. Preferably also, the cell culture medium of the invention is used for growing embryoid bodies (EBs), in particular obtained from embryonic stem cells, and differentiating the EBs into reticulocytes, enucleated cells, and/or red blood cells. As will be clear to one of skill in the art, reticulocytes, enucleated cells, and/or red blood cells can either be obtained as substantially pure cell populations or as mixtures of reticulocytes, of enucleated cells, and/or of red blood cells.

Preferably, the method of the invention is for differentiating HSCs into reticulocytes, enucleated cells, red blood, or a mixture thereof, and comprises:
- in a first step, culturing HSCs for 5 to 9, days, in particular for 7 days, in a cell culture medium comprising:
   - insulin at a concentration of 8 to 12 µg/ml;
   - transferrin at a concentration of from 300 to 350 µg/ml;
   - plasma at a concentration of from 3% to 7%;
   - heparin at a concentration of from 1.5 UI/ml to 2.5 UI/ml;
   - hydrocortisone at a concentration of from 5.10⁻⁷ to 5.10⁻⁶ M;
   - SCF at a concentration of from 80 ng/ml to 120 ng/ml;
   - IL-3 at a concentration of from 4 ng/ml to 6 ng/ml;
   - Epo at a concentration of from 2.5 to 3.5 UI/ml;
- in a second step, culturing the cells obtained in the first step for 2 to 5 days, in particular for 3 to 4 days, in a cell culture medium comprising:
   - insulin at a concentration of 8 to 12 µg/ml;
   - transferrin at a concentration of from 300 to 350 µg/ml;
   - plasma at a concentration of from 3% to 7%;
   - heparin at a concentration of from 1.5 UI/ml to 2.5 UI/ml;
   - hydrocortisone at a concentration of from 5.10⁻⁷ to 5.10⁻⁶ M;
   - SCF at a concentration of from 80 ng/ml to 120 ng/ml;
   - Epo at a concentration of from 2.5 to 3.5 UI/ml;
- in a third step, culturing the cells obtained in the second step for 6 to 10 days, in particular until day 18 to 21 from the start of the first step, in a cell culture medium comprising:
   - insulin at a concentration of 8 to 12 µg/ml;
   - transferrin at a concentration of from 300 to 350 µg/ml;
   - plasma at a concentration of from 3% to 7%;
   - heparin at a concentration of from 1.5 UI/ml to 2.5 UI/ml;
   - hydrocortisone at a concentration of from 5.10⁻⁷ to 5.10⁻⁶ M;
   - Epo at a concentration of from 2.5 to 3.5 UI/ml;
- thereby obtaining reticulocytes, enucleated cells, red blood cells, or a mixture thereof.

Preferably also, the method of the invention is for differentiating EBs into red blood cells, reticulocytes, enucleated cells, or a mixture thereof and comprises:
- in a first step, culturing EBs for 15 to 25 days, in particular for 20 days, in a cell culture medium comprising:
   - insulin at a concentration of 8 to 12 µg/ml; transferrin at a concentration of from 425 to 475 µg/ml;
   - plasma at a concentration of from 3% to 7%;
   - heparin at a concentration of from 1.5 UI/ml to 2.5 UI/ml;
   - SCF at a concentration of from 80 ng/ml to 120 ng/ml;
   - TPO at a concentration of from 80 ng/ml to 120 ng/ml;
   - FLT3 ligand at a concentration of from 80 ng/ml to 120 ng/ml;
   - BMP4 at a concentration of from 8 ng/ml to 12 ng/ml;
   - VEGF-A165 at a concentration of from 4 ng/ml to 6 ng/ml;
   - IL-3 at a concentration of from 4 ng/ml to 6 ng/ml;
   - IL-6 at a concentration of from 4 ng/ml to 6 ng/ml;
   - Epo at a concentration of from 2.5 to 3.5 UI/ml;
- in a second step, dissociating the cells obtained in the first step and culturing the dissociated cells for 6 to 10 days, in particular for 8 days, in a cell culture medium comprising:
   - insulin at a concentration of 8 to 12 µg/ml;
   - transferrin at a concentration of from 425 to 475 µg/ml;
   - plasma at a concentration of from 8% to 12%;
   - heparin at a concentration of from 2.5 UI/ml to 3.5 UI/ml;
   - SCF at a concentration of from 80 ng/ml to 120 ng/ml;
   - IL-3 at a concentration of from 4 ng/ml to 6 ng/ml;
   - Epo at a concentration of from 2.5 to 3.5 UI/ml;
- in a third step, culturing the cells obtained in the second step for 2 to 4 days, in particular for 3 days, in a cell culture medium comprising:
   - insulin at a concentration of 8 to 12 µg/ml;
   - transferrin at a concentration of from 425 to 475 µg/ml;
   - plasma at a concentration of from 8% to 12%;
   - heparin at a concentration of from 2.5 UI/ml to 3.5 UI/ml;
   - SCF at a concentration of from 80 ng/ml to 120 ng/ml;
   - Epo at a concentration of from 2.5 to 3.5 UI/ml;
- in a fourth step, culturing the cells obtained in the third step for 2 to 4 days, in particular for 3 days, in a cell culture medium comprising:
   - insulin at a concentration of 8 to 12 µg/ml;
   - transferrin at a concentration of from 425 to 475 µg/ml;
   - plasma at a concentration of from 8% to 12%;
   - heparin at a concentration of from 2.5 UI/ml to 3.5 UI/ml;
   - Epo at a concentration of from 2.5 to 3.5 UI/ml;
- in a fifth step, culturing the cells obtained in the third step for 8 to 12 days, in particular for 10 days, (i) in a cell culture medium comprising:
   - insulin at a concentration of 8 to 12 µg/ml;
   - transferrin at a concentration of from 425 to 475 µg/ml;
   - plasma at a concentration of from 8% to 12%;
   - heparin at a concentration of from 2.5 UI/ml to 3.5 UI/ml;
   - Epo at a concentration of from 2.5 to 3.5 UI/ml;
or (ii) on an adherent stromal layer;
thereby obtaining red blood cells, reticulocytes, enucleated cells, or a mixture thereof.

### EXAMPLES:

### Example 1: In vitro production of reticulocytes from hematopoietic stem cells

### Materials and Methods

### Cell culture

Normal peripheral blood mobilized with G-CSF [leukapheresis (LK) cells] was obtained from healthy donors with informed consent. CD34+ cells were isolated by supermagnetic microbead selection using Mini-MACS columns (Miltenyi Biotech, Bergisch Glodbach, Germany) (purity > 94 ± 3 %).

Cells were cultured in IMDM (Iscove modified Dulbecco's medium, Biochrom, Germany) supplemented with 2 mM L-glutamine (Invitrogen, Cergy-Pontoise, France), 330 µg/ml iron-saturated human transferrin, 10 µg/ml insulin (Sigma, Saint-Quentin Fallavier , France), 2 IU/ml heparin Choay (Sanofi, France) and 5% solvent/detergent virus inactivated (S/D) plasma. The expansion procedure comprised three steps. In the first step (days 0 - 7), 10⁴/ml CD34⁺ cells were cultured in the presence of 10⁻⁶ M hydrocortisone (HC) (Sigma), 100 ng/ml SCF (kindly provided by Amgen, Thousand Oaks, CA), 5 ng/ml IL-3 (R&D Systems, Abingdon, UK.) and 3 IU/ml Epo (Eprex, kindly provided by Janssen-Cilag, Issy-les-Moulineaux, France). On day 4, one volume of cell culture was diluted in four volumes of fresh medium containing HC, SCF, IL-3 and Epo. In the second step (3-4 days), the cells were resuspended at 10⁵/ml in fresh medium supplemented with SCF and Epo. In the third step (up to day 18-21), the cells were cultured in fresh medium in the presence of Epo alone. Cell counts were adjusted to 5x10⁵ and 1.5x10⁶ cells/ml on days 11 and 14, respectively. The cultures were maintained at 37°C in 5% CO₂ in air and results are presented in terms of the actual rate of expansion after plating.

Cells were stained with May-Grunwald-Giemsa and new methylene blue reagents (Sigma), for morphological analyses, while enucleated cells were monitored for standard hematological parameters including the MCV (fL), MCHC (%) and MCH (pg/cell) using an XE2100 automat (Sysmex, Roche Diagnostics, Basel, Switzerland).

### Flow cytometry

Cells were labeled with unconjugated or fluorescein isothiocyanate (FITC)-or phycoerythrin (PE)-conjugated antibodies. Antibodies to CD71-FITC and CD36-FITC (Becton Dickinson, San Jose, CA), glycophorin A-PE, CD45-FITC and CD34-PE (Beckman Coulter, Marseille, France) were used for phenotyping. A primary human anti-RhD antibody and a secondary goat PE-conjugated anti-human antibody (Beckman Coulter) were employed for RhD determination. Analyses were performed on a FACSCalibur flow cytometer (Becton Dickinson) using Cell Quest software.

### Deformability measurements

The reticulocytes obtained on day 18 of culture were separated from nucleated cells by passage through a deleukocyting filter (Leucolab LCG2, Macopharma, Tourcoing, France) and the enucleated cells were examined by ektacytometry, a laser diffraction method. In the ektacytometer (Technicon, Bayer Corp., Diagnostics Division, Tarrytown, NY), cells were suspended in 4% polyvinylpyrrolidone solution and exposed to an increasing osmotic gradient (60 to 450 mOsm/Kg). The change in the laser diffraction pattern of the cells was recorded. This photometric measurement produces a signal called the deformability index (DI). Analysis of the DI curve provides a measure of the dynamic deformability of the cell membrane as a function of the osmolality at a constant applied shear stress of 170 dynes/cm². DIₘₐₓ, expressed in arbitrary units and defined as the maximum value of the DI, is normally related to the mean surface area of red cells. Oₘᵢₙ defines the osmolality at which a minimum value of the DI is obtained under hypotonic conditions and depends on the initial surface/volume ratio. O_{Hyper} is the osmolality at which the DI decreases to half the value of DIₘₐₓ in the hypertonic region of the curve and is inversely related to the MCHC.

### Enzyme activities

Digitonin (0.2%) was added to erythrocytes obtained after leukocyte depletion and Hb was quantified by spectrophotometry using Drabkin's reagent. Glucose-6-phosphate dehydrogenase and pyruvate kinase activities were determined by measurement of the rate of increase in NADPH absorbance at 340 nm, using a Synchron CX4 Beckman spectrophotometer and reagents from Randox Laboratories (Crumlin, UK) and Roche Diagnostics, respectively. Results were expressed in units per gram of Hb.

### Hb analyses

Hb fractions were separated and quantified by ion exchange high performance liquid chromatography. Analyses were performed on washed cell pellets using the Bio-Rad Variant II dual program (Bio-Rad Laboratories, Hercules, CA) according to the manufacturer's instructions.

### Oxygen binding equilibria in solution

Oxygen binding curves were determined by tonometry in a 70 ml tonometer with an attached 1 cm path length cuvette. Spectral measurements were performed with a Cary 50 spectrophotometer and the temperature was controlled with a Peltier module. Analyses were carried out at 37°C in 50 mM bis-Tris (pH 7.2) containing 140 mM NaCl and 2 mM glucose. After thorough deoxygenation under nitrogen, the red cell suspensions were equilibrated at different partial pressures of oxygen by injection of known volumes of pure oxygen into the tonometer through a rubber cap using a Hamilton syringe. The fractional saturation was estimated by simulation of the absorption spectra in the visible and Soret regions as a linear combination of the fully deoxygenated and oxygenated spectra of an RBC suspension, using a least-squares fitting routine of the software Scientist (Micromath Scientific Software, Salt Lake City, UT).

### Results

### 1.1. Differentiation of hematopoietic stem cells into reticulocytes

Starting from CD34⁺ HSC derived from the peripheral blood of healthy donors after mobilization with G-CSF (LK cells), a three-step protocol in the presence of 5% solvent/detergent virus inactivated plasma (S/D plasma) was designed. Firstly, cell proliferation and erythroid commitment were induced with SCF, IL-3 and Epo for 7 days. Secondly, the erythroid proliferation was amplified with SCF and Epo for 3-4 days. In the third step, the cells were maintained until terminal maturation in the presence of Epo alone up to day 18-21. By day 18, obtained a plateau with a mean amplification of CD34⁺ cells of 66,200 ± 24,000 fold (Figure 1) was obtained and by day 18 the percentage of enucleated cells was 74 ± 5 %. At this stage, all the cells showed reticulocyte characteristics as assessed by flow analysis of polymethine dye (XE2100-Sysmex) and by new blue methylene staining. The mean cell volume (MCV) was 141 ± 6 fL, the mean corpuscular hemoglobin concentration (MCHC) 30 ± 2% and the mean cell hemoglobin (MCH) 42 ± 1 pg. Immunological characterization of this population confirmed the reticulocyte profile of the cells (Figure 2), which expressed glycophorin A (GPA), CD71 (transferrin receptor) and CD36 (platelet glycoprotein IV) at 99 ± 1 %, 44 ± 10 % and 11 ± 4 % respectively.

### 1.2. Functional analysis of the reticulocytes generated from hematopoietic stem cells

In order to perform a precise functional analysis, the reticulocytes obtained on day 18 of culture were separated from nucleated cells by passage through a deleukocyting filter (Leucolab LCG, Macopharma). These reticulocytes had a glucose-6-phosphate dehydrogenase (G6PD) content of 65 ± 3 units and a pyruvate kinase (PK) level of 94 ± 7 units per gram of hemoglobin (Hb), in keeping with the nature of a young homogenous red cell population (Jansen et al. Am J Hematol 1985; 20, 203-215). This indicates that they were capable of reducing glutathione and maintaining ATP levels, thus ensuring normal levels of 2, 3-diphosphoglycerate (2, 3-DPG).

The reticulocyte membrane deformability was analyzed by osmotic scan ektacytometry which measures erythrocyte elongation. This produces a signal called the deformability index (DI) and the maximum elongation (DIₘₐₓ) is related to the mean surface area of the cells (Clark et al. Blood 1983; 61, 899-910 et Mohandas et al. J Clin Invest 1980; 66, 563-573). The DIₘₐₓ (0.63) of the reticulocytes, which had a greater mean volume, corresponded to expected levels and confirmed the normal deformability of these cells (Figure 3). The Oₘᵢₙ of less than 80 mOsm/kg indicated an enhanced surface/volume ratio and decreased osmotic fragility of the reticulocytes, while the normal value of O_{hyper} (362 mOsm/kg) confirmed their normal hydratation. These data show that rheological properties were maintained in the cultured reticulocytes.

The reticulocytes generated *in vitro* contained adult hemoglobin A (HbA) (96 ± 0.1 %), indicating a normal process of Hb synthesis under these conditions (Figure 4).

Tonometric oxygen equilibrium measurements showed that a suspension of reticulocytes bound and released oxygen in the same manner as a suspension of native RBC. The oxygen affinity (P₅₀) was 28 mm Hg for the reticulocytes as compared to 26 ± 1 mm Hg for native RBC (Kister et al. J Biol Chem 1987; 262, 12085-12091 et Girard et al. Respir Physiol 1987; 68, 227-238), while the Hill coefficients (n₅₀) were equal to 2.4 ± 0.1 for both samples. Figure 5 represents oxygen binding isotherms at different DPG/Hb₄ ratios (from left to right: < 0.2; normal ratio ≈ 1; 2.4) simulated from oxygen binding curves using MWC model parameters (Girard et al. Respir Physiol 1987; 68, 227-238). These results indicate that levels of 2, 3-DPG in the reticulocytes are probably close to the Hb tetramer concentration, as is observed for native RBC glycolysis rates. Depletion of 2, 3-DPG as compared to a normal concentration decreases P₅₀ two fold, whereas an increase in 2, 3-DPG after prolonged incubation of RBC with 10 mM glucose raises P₅₀ by about 60 %.

### Reference Example 2: In vitro production of red blood cells from embryonic stem cells Materials and Methods

### Undifferentiated hESC cultures

The hES cell line H1 (National Institute of Health [NIH] code WA01, passages 23-45) was maintained in an undifferentiated state by weekly mechanical passage on primary mouse embryonic fibroblast (MEF) feeder cells treated with mitomycin (20µg/mL; Sigma, Saint- Quentin Fallavier, France) in knockout Dulbecco's modified Eagle's medium (DMEM, Invitrogen, Cergy Pontoise, France) supplemented with 20% knockout serum replacer (Invitrogen) and recombinant human (rhu)FGF2 (10 ng/mL; Peprotech, Neuilly-sur-Seine, France).

### Embryoid body (EB) formation

On the first day, undifferentiated hESC were treated with collagenase IV (1mg/mL; Invitrogen) and transferred to low attachment plates (Nunc, Dutscher, Brumath, France) to allow embryoid body (EB) formation during overnight incubation in differentiation medium (knockout DMEM supplemented with 20% non-heat inactivated fetal bovine serum, 1% nonessential amino acids, 1 mM L-glutamine, and 0.1 mM ß-mercaptoethanol, Invitrogen). The next day, EB were suspended in liquid culture medium (LCM) (IMDM-glutamax, Biochrom, Berlin, Germany) containing 450 µg/mL iron-saturated human transferrin (Sigma), 10 µg/mL insulin (Sigma), 5% human plasma and 2 U/mL heparin, in the presence of SCF, TPO, FLT3 ligand (100ng/mL), rhu bone morphogenetic protein 4 (BMP4; 10ng/mL), rhu VEGF-A165, IL-3, IL-6 (5ng/mL) (Peprotech) and Epo (3 U/mL) (Eprex, kindly provided by Janssen-Cilag, France) (subsequently referred to as EB medium). EB were cultured for 20 days at 37°C in a humidified 5% CO2 atmosphere, with changes of medium and cytokines every 2 or 3 days. The cells were dissociated into a single-cell suspension by incubation with collagenase B (0.4 U/mL; Roche Diagnostics, Laval, QC, Canada) for 30 min at 37°C and then cell dissociation buffer (Invitrogen) for 10 min in a 37°C water bath, followed by gentle pipetting and passage through a 70µm mesh.

### Generation of cRBC

Day 0 to day 8: Dissociated EB were counted and plated at a density of 1x10⁶ cells/mL in LCM containing 10% human plasma and 3 U/mL heparin, in the presence of SCF (100ng/mL), IL-3 (5ng/mL) and Epo (3 U/mL). On day 1, non adherent (NA) cells (4x10⁵/mL) and adherent (AD) cells (10⁶/mL) were seeded separately in the same medium and cytokines and cultured for 8 days. On day 4, one volume of cell culture was diluted in four volumes of fresh medium containing SCF, IL-3 and Epo. Day 8 to day 11: The cells were suspended at a density of 3x10⁵ (NA) or 10⁵ (AD) cells/mL and cultured in fresh medium supplemented with SCF and Epo. Day 11 to day 15: The cells were suspended at 10⁶/mL (NA and AD cells) and cultured in fresh medium supplemented with Epo. Day 15 to day 25: NA and AD cells were suspended at 2x10⁶cells/mL in LCM containing 10% human plasma and Epo, or cocultured on an adherent stromal layer. The cultures were maintained at 37 °C in 5% CO2 in air.

### Stromal cells

Three sources of adherent cell layers were evaluated : (i) the MS-5 stromal cell line, (ii) mesenchymal stromal cells (MSC) (Prockop Science 1997; 276, 71-74) established from whole normal adult bone marrow in alpha MEM (Invitrogen) supplemented with 10% fetal calf serum (FCS) (adherent MSC were expanded and purified through at least two successive passages) and (iii) stromal cells from macrophages established from CD34+ bone marrow cells in IMDM-glutamax containing 20% FCS, in the presence of SCF (50ng/mL), FLT3-ligand (30ng/mL) and TPO (15ng/mL) for 10 days and of SCF (30ng/mL), IL-3 (30ng/mL) and M-CSF (30ng/mL) for one week. FACS staining of the adherent cells was used to confirm CD14 and HLA-DR expression.

### Semisolid culture assays

BFU-E, CFU-E and CFU-GM progenitors were assayed in methylcellulose cultures. The concentration of dissociated EB was 1x10⁵cells/mL and colonies were scored on days 7 and 14 of culture.

### Flow cytometric analysis of undifferentiated hESC, EB and differentiated cells

Cells were prepared in PBS containing 0.1% BSA and labeled with a cocktail of monoclonal antibodies (mAbs). Samples were analyzed using a FACSCalibur flow cytometer with CellQuest acquisition software (Becton Dickinson, San José, CA, USA). The following antibodies were used for flow cytometric analysis of undifferentiated hESC, harvested disaggregated day 2 to day 20 EB and erythroid cells during differentiation: SSEA4-PE (phycoerythrin) and SSEA1-PE (Clinisciences, Montrouge, France); TRA-1-60, TRA-1-81, goat anti-mouse IgM-PE and goat anti-mouse IgG-PE (Chemicon, Saint-Quentin en Yvelines, France); CD34-PE, CD45-PE, CD45-PC7, CD117-PE, CD71-FITC, CD36-FITC and CD235a-PE (glycophorin A) (Beckman Coulter- Immunotech, Marseille, France); CD133-PE (Miltenyi Biotech, Glodbach, Germany). Viable cells were gated for analysis and staining with appropriate isotype-matched control mAbs was used to establish thresholds for positive staining and background.

### Hemoglobin composition of cRBC by chromatography and mass spectrometry

The percentage of the various hemoglobin fractions was measured by CE-HPLC using a Bio- Rad Variant II Hb analyzer (Bio-Rad Laboratories, Hercules, CA, USA). The separation of the different globin chains fractions contained in cRBC obtained from hES cells at D15 and D25 of culture was done by reversed phase liquid chromatography (RP-LC) and spectral analysis. RP-LC analyses were performed on a C4 Uptisphere (silica beads 5 µm; average pore size 300Å) (Interchim, Montluçon, France) (4.6 x 250 mm). Elution was obtained by a two-solvent system (A: 10% CH3CN (acetonitrile) in 0.3% TFA (trifluoroacetic acid), and B: 70% CH3CN in 0.3% TFA). The integration of the different RP-LC peaks allowed determining the area percentages of each isolated globin-chain fraction. Their identification and characterization were performed by electrospray ionizationmass spectrometry (ESI-MS) after separation and collection of of globin chains. Results were compared to data obtained with cRBC generated from human CD34+ cord blood cells.

### Functionality of hemoglobin of cRBC

The binding of hemoglobin (Hb) with carbon monoxide was studied by flash photolysis using a 4x10 mm optical cuvette (4 mm for the transmitted light and 10 mm for the laser beam). Briefly, the kinetics of CO rebinding to Hb tetramers were analyzed at 436 nm after photodissociation of the ligand with a 10-ns pulse at 532 nm (Marden et al. Biochemistry 1988; 27, 1659-1664). RBC were lysed in a hypotonic buffer solution on ice for 30 min. After centrifugation at 15,000 g, the supernatant containing the Hb was removed from the membranes and cell debris and IHP (inositol hexa-phosphate 1 mM) was added to the Hb samples. Data simulations were carried out using the non linear least-squares program of Scientist (Micromath).

### Results

### 2.1. Differentiation of hESC into hEB conditioned for erythroid commitment Establishment of the culture medium for EB

The erythropoietic pathway was induced and stimulated very early. Whereas addition of BMP4 would appear to be indispensable (Chadwick et al. Blood 2003; 102, 906-915) and likewise of VEGF-A165 (Cerdan et al. Blood 2004; 103, 2504-2512), eight different experiments were performed to test the essential role of two other parameters, cytokines and the type of serum. After carrying out these experiments, a culture medium for EB was defined (referred to as EB medium) conditioning erythroid commitment. It contains 5% pooled human plasma, a high concentration of transferrin (450 µg/mL) and a cocktail of 8 cytokines: SCF, TPO, FLT3 ligand (100 ng/mL), rhu BMP4 (10 ng/mL), rhu VEGF-A165, IL-3, IL-6 (5 ng/mL) and Epo (3 U/mL). As described in the following sections, these culture conditions allowed to obtain at the end of culture a maximum number of mature enucleated RBC.

### 2.2. Determination of the erythroid potential of hEB

First, the stage or stages of differentiation of hEB having the best erythroid potential were identified. The kinetics of differentiation of hEB between days 2 and 20 of culture following (1) the expression of specific markers of hematopoiesis and erythropoiesis by flow cytometry and (2) the formation of erythroid progenitors were analyzed. Prior to differentiation, hESC expressed high levels of markers specific for undifferentiated cells and no or low levels of hematopoietic markers. The expression of these markers of undifferentiated cells declined progressively until day 13 to remain weakly positive until day 20. CD34 was expressed from day 2 to day 20 with a peak between days 9 and 13 and CD45 from day 6 to day 20 with a peak on day 13 (Figure 7). Interestingly, the transferrin receptor CD71 was expressed throughout culture and at a high level between days 6 and 20. The erythroid markers CD36 and CD235a were weakly expressed as of day 13 (Figure 8). Overall, between days 15 and 20, hEB significantly expressed the hematopoietic and erythroid markers studied: CD45, CD34, CD71, CD36 and CD235a. Meanwhile the number of CFC remained low with a slight peak on day 15, pointing to a weak clonogenic potential of hEB (Figure 7). In view of these results, erythroid differentiation was pursued using hEB from days 15 and 20 of culture.

### 2.3. Differentiation/maturation of hEB into cRBC - Protocol for the generation of cRBC

The inventors developed simple and optimal culture conditions consisting of culture in a liquid medium in the presence of 10% human plasma and an evolving cocktail of cytokines based on SCF, IL-3 and Epo (Figure 6). In the last phase of culture as of day 15, the impact on enucleation of three different stromas known for their capacity to support hematopoiesis was tested: MS5 cells of murine origin, mesenchymal stem cells (MSC) and macrophages of human origin versus stroma-free conditions. hEB from days 15 and 20 of culture were dissociated and the cells resuspended and cultured according to the liquid culture protocol for erythroblastic differentiation/maturation. On day 1, two types of cell could be distinguished, non adherent (NA) and adherent (AD), representing respectively 10% and 90% of the total cells. The two cell types were cultured in parallel using the same protocol. Erythrocyte maturation was evaluated regularly according to the cell morphology after staining with MGG and the expression of erythroid membrane antigens as determined by flow cytometry.

### 2.4. Generation of mature enucleated cRBC starting from day 15 or day 20 hEB

The erythroid commitment of day 15 or day 20 hEB was complete after 4 days of liquid culture with production of more than 95% erythroblasts. Terminal differentiation/maturation was achieved progressively with the appearance of 3±2% enucleated cells by day 11, 17±4% by day 15, 31 ±8% by day 18 and 48±9% by day 21. At the end of culture on day 25, the population contained 58±2% perfectly enucleated RBC (Figure 9). The levels of enucleation were entirely comparable whatever the cells cultured (NA or AD), the culture conditions (with or without stroma) or the nature of the stroma (MS5, MSC or macrophages). The erythroid cells produced from day 15 or day 20 hEB were capable of generating cRBC and were called "enucleating window cells" (EWC). The only notable difference during this liquid culture phase was that the amplification of NA cells was superior to that of AD cells (24 to 61 fold *vs* 4 to 5 fold by day 20, respectively). Thus, starting from 106 cells derived from hEB, 144x106 erythroid cells, or 82x106 cRBC were generated.

### 2.5. Analysis of the cRBC generated from EWC - Membrane markers of mature cRBC

Flow cytometric analysis of the membrane antigens of the cRBC produced attested to their degree of maturity. At the end of culture, all the cRBC generated strongly expressed CD235a and CD71. The expression of CD36 decreased with increasing cell maturity (5%±1 on day 11 *vs* 7±3% on day 25), while an elevated expression of RhD antigen in more than 80% of the cells confirmed the high level of membrane maturation of the cRBC (Figure 10).

### 2.6. Size of the cRBC

At the end of liquid culture on day 25, the size of the cRBC was measured by microscopy and compared it to that of control adult RBC from peripheral blood. In the absence of stroma or after coculture on MS5 cells, MSC or macrophages, the size of the cRBC was comparable, with a mean diameter of 10 µm (Figure 11).

### 2.7. Analysis of the hemoglobin of the cRBC

To analyze the type of hemoglobin synthesized by the cRBC, a study of the globin chains by reverse phase HPLC and mass spectrometry was combined with the identification of tetrameric hemoglobin by HPLC.

### Identification and quantification of the globin chains in cRBC by RP-LC and mass spectrometry

Separation of the globin chains by RP-LC permitted quantification of the hemoglobin production of the cRBC: 1 to 5% beta, 19 to 29% gamma-G, 36 to 43% alpha and 11 to 21% gamma-A chains. Two additional peaks of variable intensity in different experiments, ranging from absent to less than 15%, were also observed and corresponded to the embryonic chains epsilon and zeta. Thus, there was a largely predominant synthesis of fetal chains (35 to 50%), a weak production of adult chains (2%) and a variable synthesis of embryonic chains (< 10%), with about 40% alpha chains. These results were confirmed by mass spectrometric identification of the fractions eluted by RP-LC and were identical to those obtained for cRBC derived from CD34+ HSC from cord blood (Figures 12 et 13).

### Study of hemoglobin synthesis by CE-HPLC

An analysis of tetrameric Hb by CE-HPLC showed the synthesis of 2.5% HbA and 74 to 80% HbF and the profiles were superimposable on those obtained for cRBC derived from CD34+ HSC from cord blood (Figures 12 et 13). These results are in agreement with our findings using RP-LC and mass spectrometry and demonstrate for the first time the synthesis of fetal hemoglobin in its tetrameric form in cRBC derived from hESC.

### 2.8. Functionality of the cRBC haemoglobin

The functionality of the cRBC hemoglobin was assessed by ligand binding kinetics after flash photolysis (Figure 14). The bimolecular kinetics after photodissociation of CO provide a sensitive probe of hemoglobin function. Thus, two phases are observed which correspond to the two hemoglobin quaternary states for ligand binding. The fast component arises from the tetramers in the R-state and the slow component from the T-state tetramers. At high levels of photodissociation the main species are mono- and doubly-liganded, while at low levels one mainly measures the CO binding to triply-liganded species. Therefore, the allosteric equilibrium for the different partially liganded species can be probed by varying the photodissociation level. The R->T transition in normal HbA occurs after binding of a second ligand to the Hb tetramer. In the presence of an allosteric effector such as IHP, the switchover point occurs later and the intrinsic R and T affinities also decrease. The CO rebinding kinetics for hemoglobin from cRBC (Figure 14) were almost superimposable on those for a sample of fetal blood, as expected from the HPLC analysis showing a large amount of HbF in the cRBC. After addition of IHP, the R->T transition was displaced towards the low affinity tetramers to a similar extent as in fetal blood (same magnitude of the slow T-state rebinding phase). The CO flash photolysis experiments thus confirmed that the HbF in cRBC is functional not only under physiological conditions but also in response to a potent allosteric effector.

## Claims

1. A cell culture medium for the growth and/or differentiation of cells of the hematopoietic lineage, comprising:
- insulin at a concentration of from 1 to 50 µg/ml;
- transferrin at a concentration of from 100 µg/ml to 2000 µg/ml;
- plasma or serum at a concentration of from 1 % to 30%;
- heparin, at a concentration 0.5 UI/ml to 5 UI/ml;
- Epo;
and optionally further comprising:
- hydrocortisone;
or
- SCF;
or
- SCF and IL-3;
or
- hydrocortisone and SCF;
or
- hydrocortisone, SCF and IL-3;
or
- SCF, TPO, FLT3, BMP4, VEGF-A165, IL-3 and IL-6.

2. The cell culture medium according to claim 1, wherein insulin is at a concentration of 8 to 12 µg/ml.

3. The cell culture medium according to claim 1 or 2, wherein transferrin is at a concentration of from 300 to 500 µg/ml.

4. The cell culture medium according to any of claims 1 to 3, wherein plasma or serum is at a concentration of from 4 to 12%.

5. The cell culture medium according to any of claims 1 to 4, wherein heparin is at a concentration of from 1.5 to 3.5 UI/ml.

6. The cell culture medium according to any of claims 1 to 5, comprising Iscove's Modified Dulbecco's Medium optionally complemented with glutamine or a glutamine-containing peptide.

7. The use of a cell culture medium as defined in any of claims 1 to 6, for the growth and/or differentiation of cells of the hematopoietic lineage in the absence of feeder cells.

8. The use according to claim 7, for growing hematopoietic stem cells (HSCs) and differentiating the HSCs into reticulocytes, enucleated cells, and/or red blood cells.

9. The use according to claim 8, for growing embryoid bodies (EBs) and differentiating the EBs into reticulocytes, enucleated cells, and/or red blood cells, in the absence of feeder cells.

10. A method for growing and/or differentiating cells of the hematopoietic lineage in the absence of feeder cells comprising at least one step of culturing cells with a cell culture medium as defined in any of claims 1 to 6.

11. The method of claim 10, for differentiating hematopoietic stem cells (HSCs) into reticulocytes, comprising:
- in a first step, culturing HSCs for 7 days in a cell culture medium comprising:
- insulin at a concentration of 8 to 12 µg/ml;
- transferrin at a concentration of from 300 to 350 µg/ml;
- plasma at a concentration of from 3% to 7%;
- heparin at a concentration of from 1.5 UI/ml to 2.5 UI/ml;
- hydrocortisone at a concentration of from 5.10⁻⁷ to 5.10⁻⁶ M;
- SCF at a concentration of from 80 ng/ml to 120 ng/ml;
- IL-3 at a concentration of from 4 ng/ml to 6 ng/ml;
- Epo at a concentration of from 2.5 to 3.5 UI/ml;
- in a second step, culturing the cells obtained in the first step for 3 to 4 days in a cell culture medium comprising:
- insulin at a concentration of 8 to 12 µg/ml;
- transferrin at a concentration of from 300 to 350 µg/ml;
- plasma at a concentration of from 3% to 7%;
- heparin at a concentration of from 1.5 UI/ml to 2.5 UI/ml;
- hydrocortisone at a concentration of from 5.10⁻⁷ to 5.10⁻⁶ M;
- SCF at a concentration of from 80 ng/ml to 120 ng/ml;
- Epo at a concentration of from 2.5 to 3.5 UI/ml;
- in a third step, culturing the cells obtained in the second step until day 18 to 21 from the start of the first step, in a cell culture medium comprising:
- insulin at a concentration of 8 to 12 µg/ml;
- transferrin at a concentration of from 300 to 350 µg/ml;
- plasma at a concentration of from 3% to 7%;
- heparin at a concentration of from 1.5 UI/ml to 2.5 UI/ml;
- hydrocortisone at a concentration of from 5.10⁻⁷ to 5.10⁻⁶ M;
- Epo at a concentration of from 2.5 to 3.5 UI/ml;
- thereby obtaining reticulocytes.

12. The method of claim 10, for differentiating embryoid bodies (EBs) into red blood cells comprising:
- in a first step, culturing EBs for 20 days in a cell culture medium comprising:
- insulin at a concentration of 8 to 12 µg/ml; transferrin at a concentration of from 425 to 475 µg/ml;
- plasma at a concentration of from 3% to 7%;
- heparin at a concentration of from 1.5 UI/ml to 2.5 UI/ml;
- SCF at a concentration of from 80 ng/ml to 120 ng/ml;
- TPO at a concentration of from 80 ng/ml to 120 ng/ml;
- FLT3 ligand at a concentration of from 80 ng/ml to 120 ng/ml;
- BMP4 at a concentration of from 8 ng/ml to 12 ng/ml;
- VEGF-A165 at a concentration of from 4 ng/ml to 6 ng/ml;
- IL-3 at a concentration of from 4 ng/ml to 6 ng/ml;
- IL-6 at a concentration of from 4 ng/ml to 6 ng/ml;
- Epo at a concentration of from 2.5 to 3.5 UI/ml;
- in a second step, dissociating the cells obtained in the first step and culturing the dissociated cells for 8 days in a cell culture medium comprising:
- insulin at a concentration of 8 to 12 µg/ml;
- transferrin at a concentration of from 425 to 475 µg/ml;
- plasma at a concentration of from 8% to 12%;
- heparin at a concentration of from 2.5 UI/ml to 3.5 UI/ml;
- SCF at a concentration of from 80 ng/ml to 120 ng/ml;
- IL-3 at a concentration of from 4 ng/ml to 6 ng/ml;
- Epo at a concentration of from 2.5 to 3.5 UI/ml;
- in a third step, culturing the cells obtained in the second step for 3 days, in a cell culture medium comprising:
- insulin at a concentration of 8 to 12 µg/ml;
- transferrin at a concentration of from 425 to 475 µg/ml;
- plasma at a concentration of from 8% to 12%;
- heparin at a concentration of from 2.5 UI/ml to 3.5 UI/ml;
- SCF at a concentration of from 80 ng/ml to 120 ng/ml;
- Epo at a concentration of from 2.5 to 3.5 UI/ml;
- in a fourth step, culturing the cells obtained in the third step for 3 days, in a cell culture medium comprising:
- insulin at a concentration of 8 to 12 µg/ml;
- transferrin at a concentration of from 425 to 475 µg/ml;
- plasma at a concentration of from 8% to 12%;
- heparin at a concentration of from 2.5 UI/ml to 3.5 UI/ml;
- Epo at a concentration of from 2.5 to 3.5 UI/ml;
- in a fifth step, culturing the cells obtained in the fourth step for 10 days, (i) in a cell culture medium comprising:
- insulin at a concentration of 8 to 12 µg/ml;
- transferrin at a concentration of from 425 to 475 µg/ml;
- plasma at a concentration of from 8% to 12%;
- heparin at a concentration of from 2.5 UI/ml to 3.5 UI/ml;
- Epo at a concentration of from 2.5 to 3.5 UI/ml;
or (ii) on an adherent stromal layer;
- thereby obtaining red blood cells.

## Patentansprüche

1. Zellkulturmedium für das Wachstum und/oder die Differenzierung von Zellen der hämatopoetischen Linie, das das Folgende umfasst:
- Insulin mit einer Konzentration von 1 bis 50 µg/ml;
- Transferrin mit einer Konzentration von 100 µg/ml bis 2000 µg/ml;
- Plasma oder Serum mit einer Konzentration von 1 % bis 30 %;
- Heparin mit einer Konzentration von 0,5 UI/ml bis 5 UI/ml;
- Epo;
und das optional ferner das Folgende umfasst:
- Hydrocortison;
oder
- SCF;
oder
- SCF und IL-3;
oder
- Hydrocortison und SCF;
oder
- Hydrocortison, SCF und IL-3;
oder
- SCF, TPO, FLT3, BMP4, VEGF-A165, IL-3 und IL-6.

2. Zellkulturmedium nach Anspruch 1, wobei das Insulin mit einer Konzentration von 8 bis 12 µg/ml vorliegt.

3. Zellkulturmedium nach Anspruch 1 oder 2, wobei das Transferrin mit einer Konzentration von 300 bis 500 µg/ml vorliegt.

4. Zellkulturmedium nach einem der Ansprüche 1 bis 3, wobei das Plasma oder Serum mit einer Konzentration von 4 bis 12 % vorliegt.

5. Zellkulturmedium nach einem der Ansprüche 1 bis 4, wobei das Heparin mit einer Konzentration von 1,5 bis 3,5 IU/ml vorliegt.

6. Zellkulturmedium nach einem der Ansprüche 1 bis 5, das Iscove's Modified Dulbecco's Medium umfasst, optional mit Glutamin oder einem glutaminhaltigen Peptid versetzt ist.

7. Verwendung eines Zellkulturmediums nach einem der Ansprüche 1 bis 6 für das Wachstum und/oder die Differenzierung von Zellen der hämatopoetischen Linie bei Abwesenheit von Fütterzellen.

8. Verwendung nach Anspruch 7 für das Ziehen von hämatopoetischen Stammzellen (HSCs) und das Differenzieren der HSCs zu Retikulozyten, enukleierten Zellen und/oder roten Blutkörperchen.

9. Verwendung nach Anspruch 8 für das Ziehen von embryonalen Körperchen (EBs) [Engl.: embryoid bodies] und das Differenzieren der EBs zu Retikulozyten, enukleierten Zellen und/oder roten Blutkörperchen bei Abwesenheit von Fütterzellen.

10. Verfahren zum Ziehen und/oder Differenzieren von Zellen der hämatopoetischen Linie bei Abwesenheit von Fütterzellen, umfassend mindestens einen Schritt der Zellkultivierung mit einem Zellkulturmedium nach einem der Ansprüche 1 bis 6.

11. Verfahren nach Anspruch 10 zum Differenzieren hämatopoetischer Stammzellen (HSCs) zu Retikulozyten, wobei das Verfahren das Folgende umfasst:
- in einem ersten Schritt, das Kultivieren der HSCs für 7 Tage in einem Zellkulturmedium, dass das Folgende umfasst:
- Insulin mit einer Konzentration von 8 bis 12 µpg/ml;
- Transferrin mit einer Konzentration von 300 bis 350 µg/ml;
- Plasma mit einer Konzentration von 3 % bis 7 %;
- Heparin mit einer Konzentration von 1,5 UI/ml bis 2,5 UI/ml;
- Hydrocortison mit einer Konzentration von 5 · 10⁻⁷ bis 5 · 10-6 M;
- SCF mit einer Konzentration von 80 ng/ml bis 120 ng/ml;
- IL-3 mit einer Konzentration von 4 ng/ml bis 6 ng/ml;
- Epo mit einer Konzentration 2,5 bis 3,5 UI/ml;
- in einem zweiten Schritt, das Kultivieren der Zellen, die in dem ersten Schritt erhalten wurden, für 3 bis 4 Tage in einem Zellkulturmedium, das das Folgende umfasst:
- Insulin mit einer Konzentration von 8 bis 12 µg/ml;
- Transferrin mit einer Konzentration von 300 bis 350 µg/ml;
- Plasma mit einer Konzentration von 3 % bis 7 %;
- Heparin mit einer Konzentration von 1,5 UI/ml bis 2,5 UI/ml;
- Hydrocortison mit einer Konzentration von 5 · 10⁻⁷ bis 5 · 10-6 M;
- SCF mit einer Konzentration von 80 ng/ml bis 120 ng/ml;
- Epo mit einer Konzentration 2,5 bis 3,5 UI/ml;
- in einem dritten Schritt, das Kultivieren der Zellen, die in dem zweiten Schritt erhalten wurden, bis zum Tag 18 bis 21 vom Beginn des ersten Schritts an, in einem Zellkulturmedium, das das Folgende umfasst:
- Insulin mit einer Konzentration von 8 bis 12 µg/ml;
- Transferrin mit einer Konzentration von 300 bis 350 µg/ml;
- Plasma mit einer Konzentration von 3 % bis 7 %;
- Heparin mit einer Konzentration von 1,5 UI/ml bis 2,5 UI/ml;
- Hydrocortison mit einer Konzentration von 5 · 10⁻⁷ bis 5 · 10-6 M;
- Epo mit einer Konzentration 2,5 bis 3,5 UI/ml;
- wodurch Retikulozyten erhalten werden.

12. Verfahren nach Anspruch 10 zum Differenzieren von embryonalen Körperchen (EBs) zu roten Blutkörperchen, wobei das Verfahren das Folgende umfasst:
- in einem ersten Schritt das Kultivieren der EBs für 20 Tage in einem Zellkulturmedium, dass das Folgende umfasst:
- Insulin mit einer Konzentration von 8 bis 12 µg/ml;
- Transferrin mit einer Konzentration von 425 bis 475 µg/ml;
- Plasma mit einer Konzentration von 3 % bis 7 %;
- Heparin mit einer Konzentration von 1,5 UI/ml bis 2,5 UI/ml;
- SCF mit einer Konzentration von 80 ng/ml bis 120 ng/ml;
- TPO mit einer Konzentration von 80 ng/ml bis 120 ng/ml;
- FLT3-Ligand mit einer Konzentration von 80 ng/ml bis 120 ng/ml;
- BMP4 mit einer Konzentration von 8 ng/ml bis 12 ng/ml;
- VEGF-A165 mit einer Konzentration von 4 ng/ml bis 6 ng/ml;
- IL-3 mit einer Konzentration von 4 ng/ml bis 6 ng/ml;
- IL-6 mit einer Konzentration von 4 ng/ml bis 6 ng/ml;
- Epo mit einer Konzentration 2,5 bis 3,5 UI/ml;
- in einem zweiten Schritt, das Dissoziieren der Zellen, die in dem ersten Schritt erhalten wurden, und das Kultivieren der dissoziierten Zellen für 8 Tage in einem Zellkulturmedium, das das Folgende umfasst:
- Insulin mit einer Konzentration von 8 bis 12 µg/ml;
- Transferrin mit einer Konzentration von 425 bis 475 µg/ml;
- Plasma mit einer Konzentration von 8 % bis 12 %;
- Heparin mit einer Konzentration von 2,5 UI/ml bis 3,5 UI/ml;
- SCF mit einer Konzentration von 80 ng/ml bis 120 ng/ml;
- IL-3 mit einer Konzentration von 4 ng/ml bis 6 ng/ml;
- Epo mit einer Konzentration 2,5 bis 3,5 UI/ml;
- in einem dritten Schritt, das Kultivieren der Zellen, die in dem zweiten Schritt erhalten wurden, für 3 Tage in einem Zellkulturmedium, das das Folgende umfasst:
- Insulin mit einer Konzentration von 8 bis 12 µg/ml;
- Transferrin mit einer Konzentration von 425 µg/ml bis 475 µg/ml;
- Plasma mit einer Konzentration von 8 % bis 12 %;
- Heparin mit einer Konzentration von 2,5 UI/ml bis 3,5 UI/ml;
- SCF mit einer Konzentration von 80 ng/ml bis 120 ng/ml;
- Epo mit einer Konzentration 2,5 bis 3,5 UI/ml;
- in einem vierten Schritt, das Kultivieren der Zellen, die in dem dritten Schritt erhalten wurden, für 3 Tage in einem Zellkulturmedium, das das Folgende umfasst:
- Insulin mit einer Konzentration von 8 bis 12 µg/ml;
- Transferrin mit einer Konzentration von 425 bis 475 µg/ml;
- Plasma mit einer Konzentration von 8 % bis 12 %;
- Heparin mit einer Konzentration von 2,5 UI/ml bis 3,5 UI/ml;
- Epo mit einer Konzentration 2,5 bis 3,5 UI/ml;
- in einem fünften Schritt, das Kultivieren der Zellen, die in dem vierten Schritt erhalten wurden, für 10 Tage (i) in einem Zellkulturmedium, das das Folgende umfasst:
- Insulin mit einer Konzentration von 8 bis 12 µg/ml;
- Transferrin mit einer Konzentration von 425 bis 475 µg/ml;
- Plasma mit einer Konzentration von 8 % bis 12 %;
- Heparin mit einer Konzentration von 2,5 UI/ml bis 3,5 UI/ml;
- Epo mit einer Konzentration 2,5 bis 3,5 UI/ml;
oder (ii) auf einer adhärenten Stromaschicht;
- wodurch rote Blutkörperchen erhalten werden.

## Revendications

1. Milieu de culture cellulaire pour la croissance et/ou la différenciation de cellules de la lignée hématopoïétique, comprenant :
- de l'insuline à une concentration comprise entre 1 et 50 µg/ml ;
- de la transferrine à une concentration comprise entre 100 µg/ml et 2000 µg/ml ;
- du plasma ou du sérum à une concentration comprise entre 1 % et 30 % ;
- de l'héparine à une concentration de 0,5 UI/ml à 5 UI/ml ;
- de l'Epo ;
et comprenant facultativement en outre :
- de l'hydrocortisone ;
ou
- du SCF ;
ou
- du SCF et de l'IL-3 ;
ou
- de l'hydrocortisone et du SCF ;
ou
- de l'hydrocortisone, du SCF et de l'IL-3 ;
ou
- du SCF, la TPO, FLT3, la BMP4, le VEGF-A165, l'IL-3 et l'IL-6.

2. Milieu de culture cellulaire selon la revendication 1, dans lequel l'insuline est à une concentration de 8 à 12 µg/ml.

3. Milieu de culture cellulaire selon la revendication 1 ou 2, dans lequel la transferrine est à une concentration comprise entre 300 et 500 µg/ml.

4. Milieu de culture cellulaire selon l'une quelconque des revendications 1 à 3, dans lequel le plasma ou le sérum est à une concentration comprise entre 4 et 12 %.

5. Milieu de culture cellulaire selon l'une quelconque des revendications 1 à 4, dans lequel l'héparine est à une concentration comprise entre 1,5 et 3,5 UI/ml.

6. Milieu de culture cellulaire selon l'une quelconque des revendications 1 à 5, comprenant un milieu de Dulbecco modifié selon Iscove facultativement complémenté avec de la glutamine ou un peptide contenant de la glutamine.

7. Utilisation d'un milieu de culture cellulaire tel que défini dans l'une quelconque des revendications 1 à 6, pour la croissance et/ou la différenciation de cellules de la lignée hématopoïétique en l'absence de cellules nourricières.

8. Utilisation selon la revendication 7, pour la croissance de cellules souches hématopoïétiques (CSH) et la différenciation des CSH en réticulocytes, cellules énucléées, et/ou globules rouges.

9. Utilisation selon la revendication 8, pour la croissance de corps embryoïdes (EB) et la différenciation des EB en réticulocytes, cellules énucléées, et/ou globules rouges, en l'absence de cellules nourricières.

10. Procédé de croissance et/ou de différenciation de cellules de la lignée hématopoïétique en l'absence de cellules nourricières comprenant au moins une étape de mise en culture des cellules avec un milieu de culture cellulaire tel que défini dans l'une quelconque des revendications 1 à 6.

11. Procédé selon la revendication 10, pour la différenciation de cellules souches hématopoïétiques (CSH) en réticulocytes, comprenant :
- dans une première étape, la mise en culture des CSH pendant 7 jours dans un milieu de culture cellulaire comprenant :
∘ de l'insuline à une concentration de 8 à 12 µg/ml ;
∘ de la transferrine à une concentration comprise entre 300 et 350 µg/ml ;
∘ du plasma à une concentration comprise entre 3 % et 7 % ;
∘ de l'héparine à une concentration comprise entre 1,5 UI/ml et 2,5 UI/ml ;
∘ de l'hydrocortisone à une concentration comprise entre 5.10⁻⁷ et 5.10⁻⁶ M ;
∘ du SCF à une concentration comprise entre 80 ng/ml et 120 ng/ml ;
∘ de l'IL-3 à une concentration comprise entre 4 ng/ml et 6 ng/ml ;
∘ de l'Epo à une concentration comprise entre 2,5 et 3,5 UI/ml ;
- dans une deuxième étape, la mise en culture des cellules obtenues dans la première étape pendant 3 à 4 jours dans un milieu de culture cellulaire comprenant :
∘ de l'insuline à une concentration de 8 à 12 µg/ml ;
∘ de la transferrine à une concentration comprise entre 300 et 350 µg/ml ;
∘ du plasma à une concentration comprise entre 3 % et 7 % ;
∘ de l'héparine à une concentration comprise entre 1,5 UI/ml et 2,5 UI/ml ;
∘ de l'hydrocortisone à une concentration comprise entre 5.10⁻⁷ et 5.10⁻⁶ M ;
∘ du SCF à une concentration comprise entre 80 ng/ml et 120 ng/ml ;
∘ de l'Epo à une concentration comprise entre 2,5 et 3,5 UI/ml ;
- dans une troisième étape, la mise en culture des cellules obtenues dans la deuxième étape jusqu'au jour 18 à 21 à partir du début de la première étape, dans un milieu de culture cellulaire comprenant :
∘ de l'insuline à une concentration de 8 à 12 µg/ml ;
∘ de la transferrine à une concentration comprise entre 300 et 350 µg/ml ;
∘ du plasma à une concentration comprise entre 3 % et 7 % ;
∘ de l'héparine à une concentration comprise entre 1,5 UI/ml et 2,5 UI/ml ;
∘ de l'hydrocortisone à une concentration comprise entre 5.10⁻⁷ et 5.10⁻⁶ M ;
∘ de l'Epo à une concentration comprise entre 2,5 et 3,5 UI/ml ;
- en obtenant ainsi des réticulocytes.

12. Procédé selon la revendication 10, pour la différenciation de corps embryoïdes (EB) en globules rouges comprenant :
- dans une première étape, la mise en culture des EB pendant 20 jours dans un milieu de culture cellulaire comprenant :
∘ de l'insuline à une concentration de 8 à 12 µg/ml ;
∘ de la transferrine à une concentration comprise entre 425 et 475 µg/ml ;
∘ du plasma à une concentration comprise entre 3 % et 7 % ;
∘ de l'héparine à une concentration comprise entre 1,5 UI/ml et 2,5 UI/ml ;
∘ du SCF à une concentration comprise entre 80 ng/ml et 120 ng/ml ;
∘ de la TPO à une concentration comprise entre 80 ng/ml et 120 ng/ml ;
∘ le ligand de FLT3 à une concentration comprise entre 80 ng/ml et 120 ng/ml ;
∘ la BMP4 à une concentration comprise entre 8 ng/ml et 12 ng/ml ;
∘ le VEGF-A165 à une concentration comprise entre 4 ng/ml et 6 ng/ml ;
∘ de l'IL-3 à une concentration comprise entre 4 ng/ml et 6 ng/ml ;
∘ de l'IL-6 à une concentration comprise entre 4 ng/ml et 6 ng/ml ;
∘ de l'Epo à une concentration comprise entre 2,5 et 3,5 UI/ml ;
- dans une deuxième étape, la dissociation des cellules obtenues dans la première étape et la mise en culture des cellules dissociées pendant 8 jours dans un milieu de culture cellulaire comprenant :
∘ de l'insuline à une concentration de 8 à 12 µg/ml ;
∘ de la transferrine à une concentration comprise entre 425 et 475 µg/ml ;
∘ du plasma à une concentration comprise entre 8 % et 12 % ;
∘ de l'héparine à une concentration comprise entre 2,5 UI/ml et 3,5 UI/ml ;
∘ du SCF à une concentration comprise entre 80 ng/ml et 120 ng/ml ;
∘ de l'IL-3 à une concentration comprise entre 4 ng/ml et 6 ng/ml ;
∘ de l'Epo à une concentration comprise entre 2,5 et 3,5 UI/ml ;
- dans une troisième étape, la mise en culture des cellules obtenues dans la deuxième étape pendant 3 jours, dans un milieu de culture cellulaire comprenant :
∘ de l'insuline à une concentration de 8 à 12 µg/ml ;
∘ de la transferrine à une concentration comprise entre 425 et 475 µg/ml ;
∘ du plasma à une concentration comprise entre 8 % et 12 % ;
∘ de l'héparine à une concentration comprise entre 2,5 UI/ml et 3,5 UI/ml ;
∘ du SCF à une concentration comprise entre 80 ng/ml et 120 ng/ml ;
∘ de l'Epo à une concentration comprise entre 2,5 et 3,5 UI/ml ;
- dans une quatrième étape, la mise en culture des cellules obtenues dans la troisième étape pendant 3 jours, dans un milieu de culture cellulaire comprenant :
∘ de l'insuline à une concentration de 8 à 12 µg/ml ;
∘ de la transferrine à une concentration comprise entre 425 et 475 µg/ml ;
∘ du plasma à une concentration comprise entre 8 % et 12 % ;
o de l'héparine à une concentration comprise entre 2,5 UI/ml et 3,5 UI/ml ;
o de l'Epo à une concentration comprise entre 2,5 et 3,5 UI/ml ;
- dans une cinquième étape, la mise en culture des cellules obtenues dans la quatrième étape pendant 10 jours, (i) dans un milieu de culture cellulaire comprenant :
∘ de l'insuline à une concentration de 8 à 12 µg/ml ;
∘ de la transferrine à une concentration comprise entre 425 et 475 µg/ml ;
∘ du plasma à une concentration comprise entre 8 % et 12 % ;
∘ de l'héparine à une concentration comprise entre 2,5 UI/ml et 3,5 UI/ml ;
∘ de l'Epo à une concentration comprise entre 2,5 et 3,5 UI/ml ;
ou (ii) sur une couche de cellules stromales adhérente ;
- en obtenant ainsi des globules rouges.
